# FASCICULE DE BREVET EUROPEEN

(11) **EP 2 406 354 B1**
(45) Date de publication et mention de la délivrance du brevet: **27.04.2016**
(21) Numéro de dépôt: 10714926.2
(22) Date de dépôt: 08.03.2010
(51) Int. Cl.: C10L 1/02, C10L 1/18, C10L 1/22, C10L 1/16, C12P 7/64, C10L 1/19, C10L 1/228, C10L 10/02

(54) **CARBURANT AVIATION CONTENANT UNE PROPORTION DE COMPOSES ORGANIQUES EX-BIOMASSE**
FLUGZEUGTREIBSTOFF MIT EINEM ANTEIL AN ORGANISCHEN VERBINDUNGEN AUS BIOMASSE
AVIATION FUEL CONTAINING A PROPORTION OF ORGANIC COMPOUNDS FROM BIOMASS

(30) Priorité: 09.03.2009 FR 0951472
(43) Date de publication de la demande: 18.01.2012
(73) Titulaire: ARKEMA FRANCE, 92700 Colombes (FR)
(72) Inventeur: DUBOIS, Jean-Luc, F-69390 Millery (FR)
(74) Mandataire: Killis, Andréas
(86) Numéro de dépôt international: PCT/FR2010/050387
(87) Numéro de publication internationale: WO 2010/103223

(56) Documents cités:
- WO-A2-2008/036654
- FR-A1- 2 894 976
- US-A- 2 135 327
- US-A1- 2008 032 913

## Description

La présente invention a pour objet un carburant aviation (ou kérosène) constitué d'un mélange de kérosène d'origine pétrolière et d'un carburant dérivé d'huiles végétales.

Les carburants aviation doivent satisfaire une série de spécifications découlant directement des conditions particulières, ils sont soumis à des variations extrêmes de température de quelque 80 °C, dans lesquelles ils sont utilisés. Dans la gamme des différents carburants actuels, le kérosène se présente comme une coupe pétrolière intermédiaire, en termes d'intervalle de distillation, entre l'essence et le gazole.
Les trois principales caractéristiques techniques du kérosène, outre son intervalle de distillation, sont la densité, la tenue au froid (point de fusion) et le point éclair. A ces caractéristiques il convient d'ajouter le contenu énergétique du carburant qui est incontournable pour la bonne exploitation de l'avion. En effet, il faut pouvoir disposer d'un carburant ayant un contenu énergétique aussi élevé que possible tout en répondant aux autres caractéristiques.

Dans le contenu énergétique on peut distinguer l'énergie spécifique et la densité énergétique. Une énergie spécifique (contenu énergétique par kilogramme) élevée est importante afin de minimiser la consommation énergétique lors du décollage lorsque les réservoirs sont pleins. Une densité énergétique (contenu énergétique par litre) élevée est importante afin de minimiser les volumes de réservoirs, car tout volume supplémentaire implique aussi une surcharge pondérale pour la structure de l'avion et des réservoirs.

L'industrie aéronautique est un secteur en très forte croissance dont l'exploitation est dépendante de l'approvisionnement en carburant dont l'unique source est le pétrole. Ce combustible étant en voie de disparition à l'horizon de quelques décennies, les industriels ont lancé depuis quelques années des études pour trouver des solutions de substitution.
L'article de Georges Marsh « Biofuels : aviation alternative » paru dans « renewable energy focus » July/August 2008 pages 48 à 51 donne un large panorama des voies qui sont explorées actuellement. On peut ajouter pour être complet sur l'avenir de l'aviation des réflexions sur l'utilisation d'un combustible d'une autre nature tel que l'hydrogène. Cependant cette dernière voie impliquerait la conception de nouveaux moteurs et donc d'une mutation totale de cette industrie. Les voies qui prévalent aujourd'hui sont celles que les anglo-saxons nomment le « drop-in » c'est à dire qui proposent des carburants « synthétiques compatibles avec le kérosène actuel et donc pouvant être utilisés en mélange avec ce dernier avec pour objectif, à terme, de le remplacer complètement.

Ces travaux qui ont pour objectif la fabrication d'un nouveau carburant renouvelable i.e. non fossile s'inscrivent aussi dans la préoccupation actuelle de limitation de rejet de CO₂ dans l'atmosphère ou plus exactement, dans la limitation de l'accroissement des émissions de CO₂ dans l'atmosphère.
Parmi les voies d'investigation, on peut citer les fuels synthétiques qui consistent à transformer chimiquement le charbon (CTL pour coal to liquid selon la terminologie usuelle) ou de gaz (GTL pour gas to liquid) selon la synthèse dite Fischer-Tropsch. Cette voie de synthèse est connue depuis fort longtemps et est opérationnelle sur le plan industriel. Son avantage est que le carburant de synthèse, hydrocarbure, est parfaitement compatible avec le kérosène. Ses inconvénients sont cependant multiples. Tout d'abord le coût de la synthèse est élevé. Ensuite, le procédé de synthèse est coûteux en énergie ce qui entraîne une production importante de CO₂ préjudiciable. Enfin, la matière première étant d'origine fossile on ne peut pas parler de source de carburant renouvelable.
La préoccupation principale étant de trouver à terme un substitut permanent au pétrole, il est nécessaire que la source (ou le gisement) de ce carburant soit renouvetable ce qui conduit à rechercher des produits de culture riches en carbone qui vont constituer la biomasse. Pour aller plus avant dans cette voie on doit prendre en compte un paramètre supplémentaire important à savoir que ce produit de culture ou la culture de ce produit n'ait pas d'incidence sur le développement de la culture destinée à l'alimentation humaine indispensable avec l'évolution de la population mondiale.
Pour résumer la sélection de l'une de ces voies dépend naturellement des propriétés «thermiques et énergétiques» des carburants ainsi obtenus mais aussi de trois critères additionnels importants à savoir, la compatibilité de ces carburants avec les moteurs actuellement utilisés, le respect des nouvelles normes en matière d'émission de CO₂ et l'harmonisation (non concurrence) avec les cultures dédiées à l'alimentation. L'application du premier critère conduit à laisser provisoirement de côté l'hydrogène qui impliquerait la mise au point de nouveaux réacteurs, celui du deuxième les carburants synthétiques à base de carbone fossile en raison de la production de CO₂ et celui du troisième une sélection parmi les plantes riches en carbone destinées à la production de une biomasse énergie non concurrente de l'alimentation.

Actuellement il n'existe pas vraiment de solution pour fabriquer un carburant aviation dérivé de la biomasse satisfaisant aux réglementations en place et utilisable en quantités importantes (supérieures à 10 % volume) en mélange avec le kérosène.

Dans la voie biomasse il existe bien entendu la voie alcool représentée par l'éthanol et le méthanol qui sont obtenus par fermentation de sucres. Des carburants à base de tels alcools ont été utilisés notamment au Brésil pour les véhicules terrestres. Ils l'ont été également pour de l'aviation légère mais en raison de leurs caractéristiques, ils ne sont absolument pas adaptés à la fabrication de compléments ou de substituts du kérosène.

Des travaux plus importants ont été conduits en utilisant les connaissances développées dans le cadre du biodiesel carburant substitut du gazole pour les moteurs diesel. La base de ces travaux a été l'utilisation des huiles végétales obtenues à partir de graines de diverses plantes telles que le colza, soja, le tournesol...qui sont constituées d'un mélange de triglycérides d'acides organiques gras dont la longueur de chaîne est généralement comprise entre 16 et 18 atomes de carbones.
Dans ce cadre, un biocarburant pour moteur diesel a été développé qui est connu comme l'Ester Méthylique d'Huile Végétale (EMHV), dont une molécule majoritaire est l'ester méthylique d'acide oléique. Les acides gras sont obtenus à partir des graines de ces plantes dont sont extraits leurs triglycérides qui, par hydrolyse, fournissent les acides gras correspondants qui sont ensuite estérifiés avec le méthanol. Les esters peuvent être obtenus, par transestérification directe de l'huile végétale en présence de méthanol qui conduit à un mélange d'esters. De manière analogue, on a préparé l'EEHV c'est à dire l'ester éthylique correspondant essentiellement représenté par l'ester éthylique d'acide oléique. Ces biodiesels sont connus en anglais sous les noms de FAME et FAEE pour respectivement « Fatty. Acid Methyl Ester» et « Fatty Acid Ethyl Ester». Ils forment ce qu'on appelle les biodiesels de première génération.
On connaît également les biodiesels de deuxième génération qui sont obtenus par hydrotraitement des huiles végétales conduisant par hydrogénation à des hydrocarbures à longue chaîne, isomérisés ou non. L'isomérisation des paraffines permet de diminuer significativement le point de trouble, c'est à dire la température à laquelle les paraffines commencent à cristalliser.
Il a également été proposé, dans un article de N.M. Irving publié dans 16th European Biomass Conference, 2-6 Juin 2008, Valence, Espagne sous le titre « Clean, High Enthalpy biofuels » de substituer par nitrilation une fonction nitrile à la fonction acide de l'acide gras. Ces nitriles gras obtenus directement à partir d'acides gras naturels obtenus par hydrolyse des triglycérides contenus dans les graines, comportant des chaînes comprises entre C12 et C18 et centrées principalement sur C16 semblent donner d'excellents résultats dans l'application biodiesel.

Les acides gras estérifiés ou transformés, tels que l'EMHV, que l'on connaît classiquement comme biodiesels constituent une coupe dont les points d'ébullition d'une part sont trop élevés et les points de fusion d'autre part également trop élevés, ces caractéristiques étant liés directement à la longueur de chaîne de l'acide gras, classiquement de 16 à 18 atomes de carbone, pour pouvoir être incorporés en quantités importantes dans les coupes de carburant aviation. FR 2 894 976 décrit une composition d'essence aviation comprenant des esters choisis parmi les alkyls carboxylate.
Une solution pourrait consister à utiliser des huiles végétales correspondant à des chaînes grasses plus courtes, telles que les huiles de coco, de palmiste ou de cuphéa par exemple. Cependant, les esters de ces huiles végétales ne satisfont pas alors le critère de densité (masse volumique) car ils sont trop denses par rapport aux carburants actuels et ne répondent pas non plus aux spécifications en matière de contenu énergétique (énergie spécifique). Un autre inconvénient majeur pour l'utilisation de ces huiles est leur coût relativement élevé.

Le problème a résoudre est donc de trouver un carburant basé sur une source renouvelable, et satisfaisant au mieux aux critères (spécifications) des carburants aviation et notamment aux critères de densité, de points d'ébullition et de fusion et de contenu énergétique.

L'invention vise à pallier ces inconvénients en proposant des carburants aviation constitués d'un mélange d'un kérosène issu du pétrole et de composés choisis parmi les nitriles et les esters d'acides gras moyens obtenus à partir d'acides gras mono-insaturés d'origine naturelle renouvelables, ces acides gras moyens comportant de 7 à 12 atomes de carbone par molécule.

Il a été en effet découvert que les nitriles gras ainsi que les esters gras, à longueur de chaîne moyenne, grâce à leurs caractéristiques de densité, de températures d'ébullition et de fusion et de contenu énergétique, pouvaient constituer une coupe particulièrement intéressante comme carburant aviation.

Dans la suite de la présente description, il sera mentionné la mise en oeuvre comme matière première d'acides gras mono insaturés éventuellement hydroxylés, naturels et renouvelables. Ce terme d'acide gras englobe aussi bien l'acide proprement dit, sa forme ester ainsi que sa forme nitrile. Le passage d'une forme acide à une forme ester est parfaitement banale; le passage de la forme acide à la forme nitrile est lui aussi bien connu ainsi que cela sera explicité plus avant dans la description. Il est même parfaitement possible de passer à cette forme nitrile par traitement direct des triglycérides contenus dans les huiles ou graisses naturelles.

L'invention a pour objet un carburant aviation comprenant de 20 à 99 % poids d'une fraction d'un composé, obtenu par transformation chimique à partir d'acides gras mono insaturés éventuellement hydroxylés, naturels et renouvelables de longueur de chaîne au moins égale à 14 atomes de carbone, choisi parmi les nitriles et les esters d'acides gras moyens comportant de 7 à 12 atomes de carbone par molécule et de 1 à 80 % poids d'un kérosène issu du pétrole répondant aux spécifications mondiales des carburants aviation comme dans la revendication 1.
Par spécifications mondiales des carburants aviation il faut entendre l'ensemble des spécifications définies dans le document « World Jet Fuel Spécifications with Avgas Supplement » Edition 2005 publié par EXXON-MOBIL. Il couvre aussi bien les carburants de type Jet (Aviation Turbine Fuel -Jet A et Jet A1) que les « Military Turbine Fuel Grades » et les « Aviation Gasolines ». En effet, ces carburants de type kérosène présentent la particularité de devoir répondre dans le monde entier strictement aux mêmes niveaux de. spécifications afin d'assurer la sécurité dés transports effectués.
Cette fraction du composé représentera 20 à 99 % poids et le kérosène répondant aux spécifications mondiales de 1 à 80 % poids.
Le choix des teneurs respectives dépendra des conditions technico-économiques . d'approvisionnement en chacun des constituants. Cependant, on peut observer que certains composés ne répondent pas, pris isolément, aux spécifications mondiales, ce qui impose leur utilisation en mélange avec un kérosène ex pétrole avec des proportions dictées par l'écart entre les caractéristiques du composé et les spécifications.
Dans une autre variante préférée de l'invention, la fraction du composé sera constituée d'un mélange'd'au moins un des composés nitrile et/ou ester et d'au moins une oléfine et/ou alcane comportant de 6 à 13, de préférence 7 à 12 atomes de carbone par molécule, d'origine naturelle, issus de transformations chimiques analogues à celle appliquée aux-dits acides gras mono insaturés éventuellement hydroxylés, naturels et renouvelables. La part de l'oléfine et/ou alcane dans le mélange constituant la fraction du composé représentera de 10 à 50 % poids du mélange.

En effet, certaines molécules, alcanes ou alcènes d'une longueur de chaîne sensiblement analogue à celle des composés nitriles et/ou esters, c'est à dire comportant de 6 à 13 atomes de carbone par molécule peuvent, si on les introduit dans le mélange carburant, compenser les « insuffisances » du composé. Cette variante de réalisation pourrait même permettre à terme de s'affranchir de l'utilisation du kérosène. Cette voie pourrait sur le plan économique devenir viable notamment dans le cas où les traitements chimiques appliqués aux acides gras naturels comportant au moins 14 atomes de carbone permettraient, outre celle du composé nitrile et/ou ester la synthèse de tels alcènes et/ou alcanes.
Il est à noter que ces alcanes ou oléfines se trouveront dans le carburant aviation final aux côtés de molécules analogues provenant du kérosène. Il sera cependant possible de distinguer les unes des autres en raison de l'origine des hydrocarbures renouvelables qui comporteront une fraction de ¹⁴C_{.}

Dans un mode de réalisation préféré, l'aldéhyde est converti en acétal, ou acide, ester, nitrile alcool ou alcane ou oléfine.

Les composés sous leur forme esters d'acides gras moyens comporteront un nombre impair d'atomes de carbone, 7, 9 ou 11. Les esters constitués de 10 ou 12 atomes de carbone seront sous la forme d'un ester ω-insaturé.
Les composés sous leur forme nitrile seront sous une forme saturée ou mono-insaturee. Le nitrile gras comportant 12 atomes de carbone sera de préférence ω-insaturé.
Ces nitriles gras pourront comporter, selon certains processus de fabrication, outre la fonction nitrile, une autre fonction, soit ester, soit nitrile. Ces composés pourront être introduits dans le carburant si leurs caractéristiques, densité, températures de changement de phase et contenu énergétique sont suffisantes pour ne pas avoir à les soumettre à une nouvelle et coûteuse transformation.
Le radical alcool entrant dans les fonctions esters comportera de 1 à 4 atomes de carbone. De préférence ce radical sera méthyle pour conférer au composé ses meilleures propriétés dans les carburants de l'invention.

Leurs caractéristiques physiques des différentes molécules susceptibles d'entrer dans la composition du carburant aviation de l'invention sont données dans les tableaux 1 à 3 ci-dessous qui se rapportent aux nitriles (Tableau 1), aux esters ou nitrile-esters (Tableau 2) et oléfines et alcanes (Tableau 3).

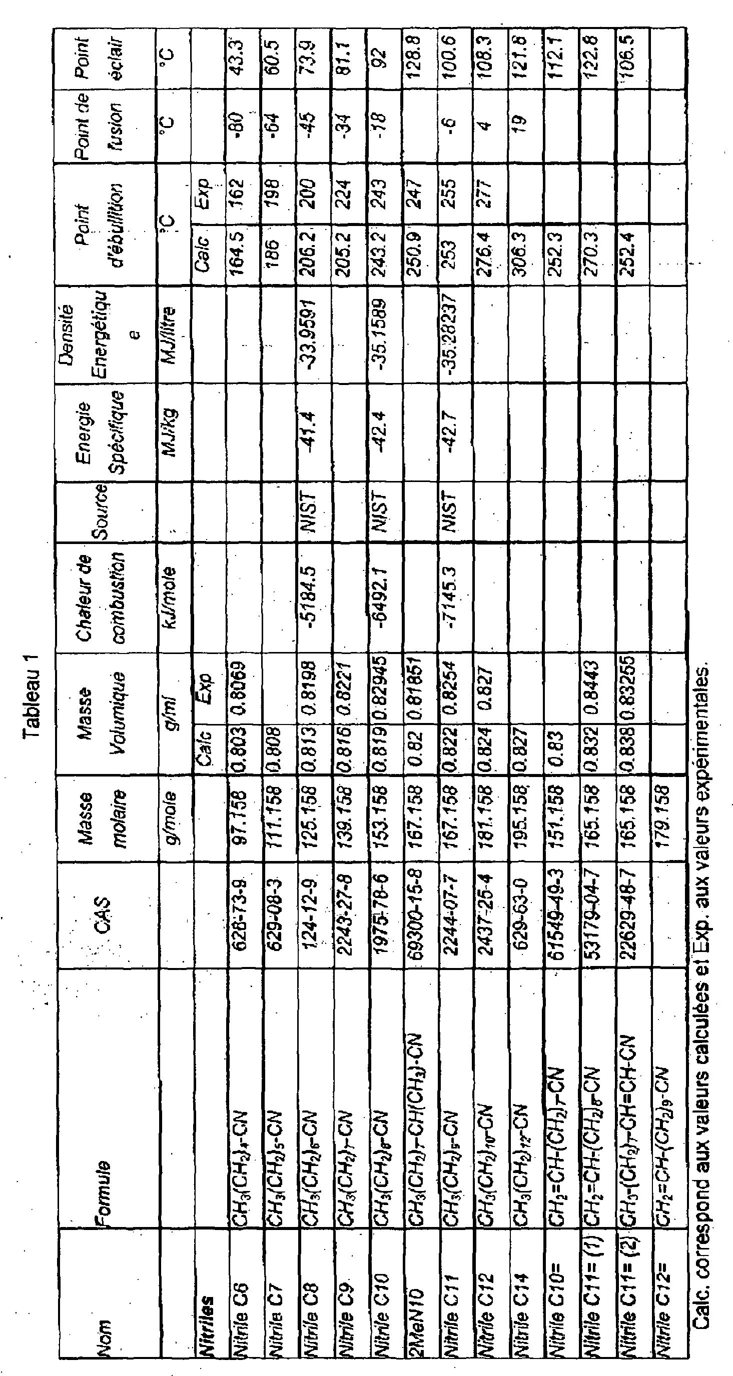

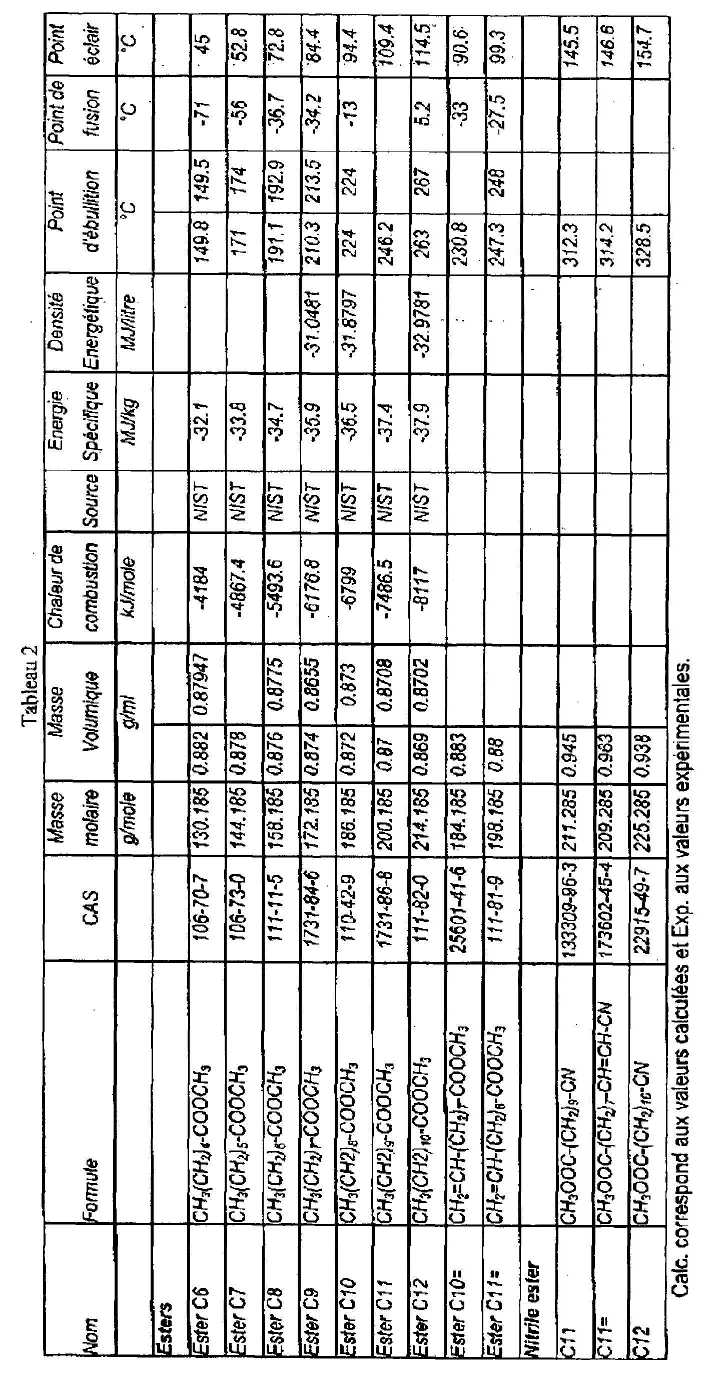

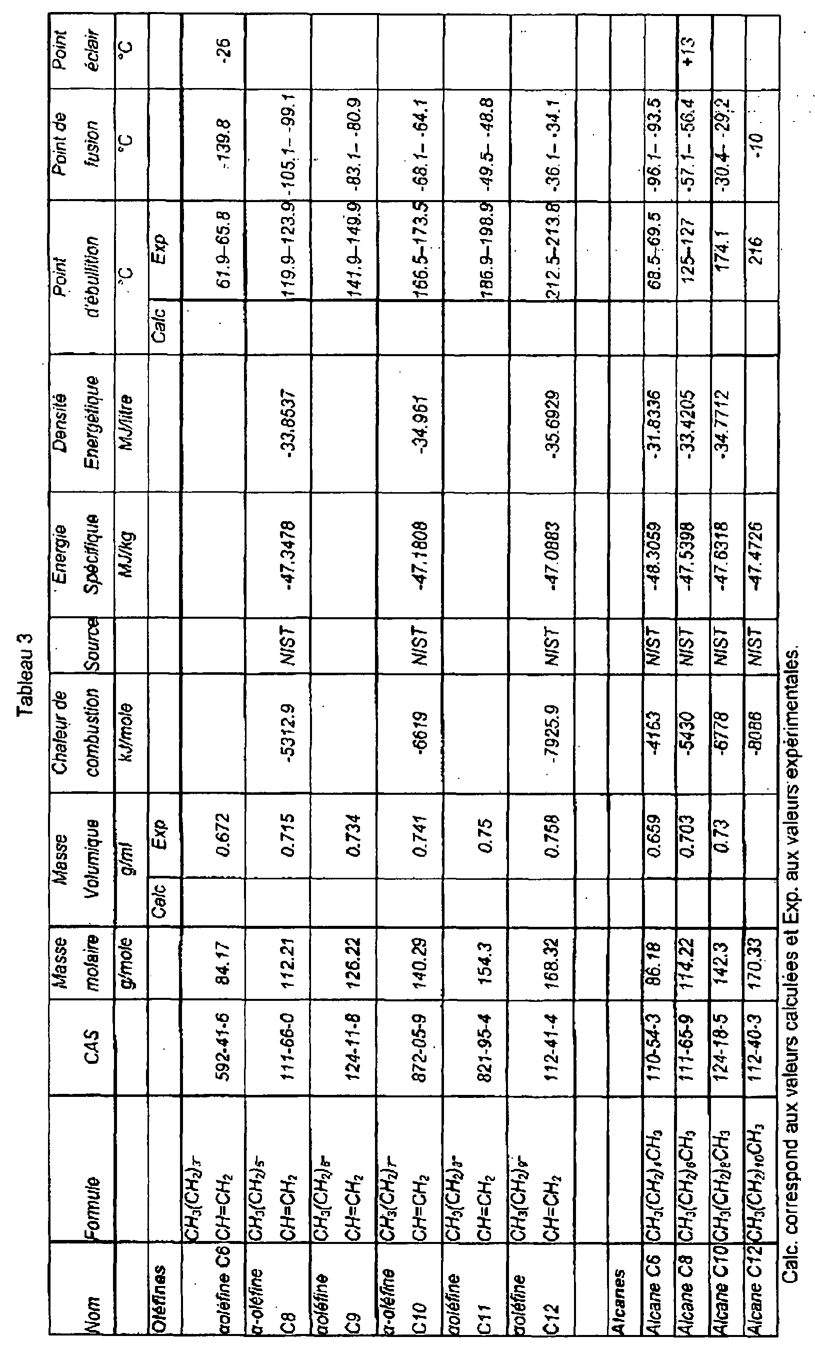

On peut observer que les α-oléfines et alcanes présentent certaines caractéristiques : point de fusion très bas, densité faible, énergie spécifique élevée qui peuvent constituer une excellente source pour être incorporée en complément du mélange dans une coupe kérosène. Ils permettent notamment de compenser les défauts de certains composés tels que les esters en matière de densité et d'énergie spécifique. Il y a donc un avantage particulier à garder en mélange les oléfines et les esters insaturés produits lors de la transformation chimique de l'acide gras d'origine, d'hydrogéner en même temps les oléfines et les esters insaturés pour donner un mélange alcanes-esters saturés. L'association d'un composé nitrile et d'une oléfine ou d'un alcane conférera au mélange les mêmes avantages. La production de ces deux éléments peut intervenir au cours du même processus réactionnel des lors que l'acide gras traité est sous la forme nitrile.
Cette solution est plus avantageuse que la solution habituelle qui consiste à hydrogéner totalement les huiles végétales en ce qu'elle consomme moins d'hydrogène, et contribue aussi à un abaissement des points d'ébullition et des températures de solidification.

L'ensemble des composés nitriles présentent une densité parfaitement adaptée à l'utilisation. En ce qui concerne le contenu énergétique, on peut observer que le pouvoir calorifique (énergie spécifique) exprimé en MJ/kg pour la quasi totalité de ces nitriles est tout à fait analogue à celui de norme Jet A1. Dans le domaine des températures : ébullition, fusion et point éclair, ces composés sont globalement à l'intérieur des normes. En ce qui concerne les esters, leurs caractéristiques même si elles sont moins bonnes permettent leur introduction en quantités mineures dans les carburants de l'invention. Leur longueur de chaîne plus courte que celle des esters d'acides gras naturels des huiles conventionnelles (soja, colza, tournesol et même Jatropha) les rendent beaucoup plus compatibles avec une application Jet Fuel, tant du point de vue du point de fusion (point de trouble) que des points d'ébullition par exemple.

Les composés de type ester et/ou nitrile entrant dans la constitution du carburant sont obtenus à partir d'acides gras mono-insaturés d'origine naturelle et renouvelable. Ces acides gras sont des acides supérieurs c'est à dire comportant au moins 14 atomes de carbone par molécule. Ces acides gras sont notamment les acides myristoléique (14C=), palmitoléique (16C=), pétrosélinique (18C=), oléique (18C=), vaccénique (18C=), gadoléique (C20=), cétoléique (22C=) et érucique (22C=) ainsi que les acides gras mono-hydroxylés, ricinoléique (18C=OH) et lesquérolique (20C=OH).
Les composés entrant dans la constitution du carburant aviation de l'invention sont obtenus à partir des huiles (ou graisses) naturelles qui sont traitées par hydrolyse ou méthanolyse pour obtenir les acides gras ou leurs esters méthylique en mélange avec le glycérol dont ils sont séparés avant traitement chimique. On peut aussi former les nitriles gras directement à partir des huiles végétales. Il est particulièrement avantageux de former le nitrile à partir de l'huile lorsque celle-ci est riche en acides gras libres comme dans le cas des huiles naturelles de cuisson usagées, ou celui de certaines-huiles naturelles. Pour l'obtention des nitriles on préfère utiliser les acides gras comme matière première.
Les transformations des acides/esters/nitriles gras mono-insaturés à longue chaîne sont fondées sur un fractionnement de la molécule à longue chaîne au niveau de la double liaison. Ce fractionnement sera effectué soit par une réaction de métathèse croisée avec l'éthylène (éthénolyse), soit par une coupure oxydante de la double liaison sous l'action d'un oxydant fort et notamment par ozonolyse oxydante, soit par craquage thermique pour les acides gras hydroxylés.
La réaction d'éthénolyse s'accompagne souvent d'une isomérisation du substrat et/ou des produits, ce qui a pour effet de conduire à un mélange de produits. Cependant dans l'utilisation présente, le mélange de produits est tout à fait compatible avec l'application visée ce qui dispense de la recherche d'un catalyseur qui ne serait pas du tout isomérisant. L'isomérisation, dans le cas de l'éthénolyse de l'ester de l'acide oléique par exemple, a pour effet de conduire à la formation d'un mélange d'esters autour de 10 carbones et d'un mélange d'oléfines aussi autour de 10 carbones. Lorsque dans la suite de la présente demande, il sera indiqué qu'un carburant comprend, à côté du kérosène, x % d'un composé nitrile ou ester (obtenu par l'intermédiaire d'une métathèse) de « n » atomes de carbone, cela signifiera que ce composé sera un mélange constitué de nitriles ou esters comportant de n-2 à n+2 atomes de carbone avec une majorité de composés à n atomes de carbone.
Dans une variante du procédé de fractionnement, la métathèse croisée peut être réalisée avec l'acrylonitrile avec formation à côté du nitrile α-insaturé de difonctionnels nitrile-esters .
A titre d'illustration des divers traitements de transformation de ces acides gras à longue chaîne en composés ester et/ou nitrile de l'invention, on donnera quelques exemples de processus.

L'acide myristoléique est soumis à une éthénolyse (métathèse croisée avec l'éthylène) selon la réaction suivante (les formules sont exprimées en forme acide par souci de simplification de l'exposé même si la réaction met en jeu l'ester ou le nitrile) :

CH₃-(CH₂)₃-CH=CH-(CH₂)₇-COOH + CH₂=CH₂→ CH₃-(CH₂)₃-CH=CH₂ + CH₂=CH-(CH₂)₇-COOH

Dans une deuxième étape l'α-oléfine en C6 est éventuellement soumise à une réaction de métathèse croisée avec l'acrylo-nitrile pour obtenir un nitrile insaturé en C7 selon la réaction :

CH₃-(CH₂)₃-CH=CH₂ + CH₂=CH-CN → CH₃-(CH2)₃-CH=CH-CN + CH₂=CH₂

Par hydrogénation on obtient le nitrile saturé en C7.

Comme cela a été indiqué précédemment, l'oléfine en C6 pourra être introduite dans le mélange carburant, elle est aussi compatible avec l'application, et peut être hydrogénée en hexane, lui aussi compatible.
Par ailleurs le second produit (acide) de la réaction après estérification forme un ester ω-insaturé en C10 utilisable comme tel (ou sa forme saturée par hydrogénation) mais qui peut également être transformé par métathèse croisée avec l'acrylonitrile selon une réaction analogue à celle décrite ci-dessus pour l'α-oléfine en C6, en un nitrile-ester insaturé, en C11
Il est également possible de procéder au préalable (avant éthénolyse) à la nitrilation de l'acide myristoléique qui conduit par éthénolyse à la formation du nitrile ω-insaturé en C10, à côté de l'oléfine.

L'acide palmitoléique est soumis à une éthénolyse selon la réaction suivante

CH₃-(CH₂)₅-CH=CH-(CH₂)₇-COOH + CH₂=CH₂ → CH₃-(CH₂)₅-CH=CH₂ + CH₂=CH-(CH₂)₇-COOH

Dans une deuxième étape l'α-oléfine en C8 est éventuellement soumise à une réaction de métathèse croisée avec l'acrylonitrile pour obtenir un nitrile insaturé en C9 selon la réaction

CH₃-(CH₂)₅-CH=CH₂ + CH₂=CH-CN → CH₃-(CH₂)₅-CH=CH-CN + CH₂=CH₂

Par hydrogénation on obtient le nitrile saturé en C9.
Comme cela a été indiqué précédemment, l'oléfine en C8 pourra être incorporée dans le mélange carburant. Elle pourra auparavant être hydrogénée.
L'acide issu de la réaction d'éthénolyse conduit au même ester ou nitrile-ester que dans le cas de l'acide myristoléique.
Comme pour l'acide myristoléique,' une nitrilation préalable à l'éthénolyse de l'acide conduit à la formation du nitrile ω-insaturé en C10.

L'acide pétrosélinique est soumis à une éthénolyse selon la réaction suivante

CH₃-(CH₂)₁₀-CH=CH-(CH₂)₄-COOH + CH₂=CH₂ → CH₃-(CH₂)₁₀-CH=CH₂ + CH₂=CH-(CH₂)₄-COOH

L'oléfine insaturée en C13, peut être incorporée telle quelle en mélange avec l'ester
L'acide ω-insaturé en C7 conduit par estérification à l'ester correspondant. En outre en appliquant à ce dernier une réaction de métathèse avec l'acrylonitrile on obtient un nitrile-ester insaturé en C8. En effectuant une métathèse croisée avec l'acrylate de méthyle on obtient un ester insaturé en C8:
La nitrilation préalable de l'acide conduit par éthénolyse au nitrile ω-insaturé en C7.

L'acide oléique est soumis à une éthénolyse selon la réaction suivante

CH₃-(CH2)₇-CH=CH-(CH₂)₇-COOH + CH₂=CH₂ → CH₃-(CH₂)₇-CH=CH₂ + CH₂=CH-(CH₂)₇-COOH

L'α-oléfine insaturée en C10 est éventuellement soumise à une réaction de métathèse croisée avec l'acrylonitrile pour obtenir un nitrile insaturé en C11 selon la réaction

CH₃-(CH₂)₇-CH=CH₂ + CH₂=CH-CN → CH₃-(CH₂)₇-CH=CH-CN + CH₂=CH₂

Par hydrogénation on obtient le nitrile saturé en C11.
Comme indiqué précédemment, l'oléfine en C10 pourra être incorporée dans le mélange carburant. Elle pourra auparavant être hydrogénée.
L'acide ω-insaturé en C10 issu de l'éthénolyse peut être estérifié. Une hydrogénation de l'ester ainsi obtenu permet d'obtenir l'ester en C10. En outre, en appliquant à cet ester ω-insaturé une réaction de métathèse avec l'acrylonitrile on obtient un nitrite-ester insaturé en C11.
La nitrilation préalable de l'acide conduit par éthénolyse au nitrile ω-insaturé en C10.

L'acide oléique peut également être soumis à une coupure oxydante telle qu'une ozonolyse oxydante selon la réaction (simplifiée) suivante (l'ozonolyse oxydante fait appel à une source d'oxygène après la formation de l'ozonide pour que la décomposition de celui ci conduise à deux groupements acides)

CH₃-(CH₂)₇-CH=CH-(CH₂)₇-COOH + O₃ (O₂)→ CH₃-(CH₂)₇-COOH + HOOC-(CH₂)₇-COOH

Après estérification du monoacide, on obtient l'ester en C9.

L'acide vaccénique est soumis à une éthénolyse selon la réaction suivante

CH₃-(CH₂)₅-CH=CH-(CH₂)₉-COOH + CH₂=CH₂ → CH₃-(CH₂)₅-CH=CH₂ + CH₂=CH-(CH₂)₉-COOH

L'α-oléfine insaturée en C8 est éventuellement soumise à une réaction de métathèse croisée avec l'acrylonitrile pour obtenir un nitrile insaturé en C9 selon la réaction

CH₃-(CH₂)₅-CH=CH₂ + CH₂=CH-CN → CH₃-(CH₂)₅-CH=CH-CN + CH₂=CH₂

Par hydrogénation on obtient le nitrile en C9.
Comme indiqué précédemment, l'oléfine en C10 pourra être incorporée dans le mélange carburant. Elle pourra auparavant être hydrogénée.
L'acide ω-insaturé en C12 issu de l'éthénolyse peut être estérifié pour obtenir l'ester ω-insaturé en C12.
La nitrilation préalable de l'acide conduit par éthénolyse au nitrile ω-insaturé en C12.

L'acide vaccénique peut également être soumis à une coupure oxydante telle qu'une ozonolyse oxydante selon la réaction (simplifiée) suivante

CH₃-(CH₂)₅-CH=CH-(CH₂)₉-COOH + O₃ (O₂) → CH₃-(CH₂)₅-COOH + HOOC-(CH₂)₉-COOH

Après estérification, on obtient d'une part l'ester en C7 et d'autre part le diester en C11. Ce diester C11 peut être nitrilé soit totalement pour former un dinitrile soit partiellement pour parvenir au nitrile ester. On peut aussi former le dinitrile à partir du diacide.

L'acide gadoléique est soumis à une éthénolyse selon la réaction suivante

CH₃-(CH₂)₉-CH=CH-(CH₂)₇-COOH + CH₂=CH₂ → CH₃-(CH₂)₉-CH=CH₂ + CH₂=CH-(CH₂)₇-COOH

La nitrilation préalable de l'acide conduit par éthénolyse au nitrile ω-insaturée en C10:
L'α-oléfine insaturée en C12 éventuellement soumis à coupure oxydante telle qu'une ozonolyse selon la réaction (simplifiée) suivante

CH₃-(CH₂)₉-CH=CH₂ + O₃ (O₂)→ CH₃-(CH₂)₉-COOH + HCOOH. L'acide en C11 issu de

l'ozonolyse est estérifié en ester C11 et peut, le cas échéant être transformé en nitrile C11.
Comme indiqué précédemment, l'oléfine en C12 pourra être incorporée dans le mélange carburant. Elle pourra auparavant être hydrogénée.
L'acide ω-insaturé en C10 est estérifié, puis le cas échéant hydrogéné en ester C10 saturé, la fonction ester ou acide, dans l'un et l'autre cas, pouvant être transformée en nitrile.
Cet acide, après estérification au méthanol par exemple, peut également être soumis à une réaction de métathèse croisée avec l'acrylonitrile pour obtenir un nitrile-ester insaturé en C11 selon la réaction

CH₂=CH-(CH₂)₇-COOMe + CH₂=CH-CN → CN-CH=CH-(CH₂)₇-COOMe + CH₂=CH₂

Par hydrogénation on obtient le nitrile-ester en C11.

L'acide cétoléique est soumis à une éthénolyse selon la réaction suivante

CH₃-(CH₂)₉-CH=CH-(CH₂)₉-COOH + CH₂=CH₂ → CH₃-(CH₂)₉-CH=CH₂ + CH₂=CH-(CH₂)₉-COOH

La nitrilation préalable de l'acide conduit par éthénolyse au nitrile ω-insaturé en C12.

L'α-oléfine insaturée en C12 soumis éventuellement à une coupure oxydante telle qu'une ozonolyse selon la réaction (simplifiée) suivante

CH₃-(CH₂)₉-CH=CH₂ + O₃ (O₂)→ CH₃-(CH₂)₉-COOH + HCOOH.

L'acide en C11 est estérifié en ester C11 qui peut, le cas échéant être transformé en nitrile C11. L'acide ω-insaturé en C12 est estérifié, puis le cas échéant hydrogéné en ester C12 saturé, la fonction ester ou acide, dans l'un et l'autre cas, pouvant être transformée en nitrile.

L'acide érucique est soumis à une éthénolyse selon la réaction suivante

CH₃-(CH₂)₇-CH=CH-(CH₂)₁₁-COOH + CH₂=CH₂ → CH₃-(CH₂)₇-CH=CH₂ + CH₂=CH-(CH₂)₁₁-COOH

L'α-oléfine insaturée en C10 est soumise éventuellement à une réaction de métathèse croisée avec l'acrylonitrile pour obtenir un nitrile insaturé en C11 selon la réaction

CH₃-(CH₂)₇-CH=CH₂ + CH₂=CH-CN → CH₃-(CH₂)₇-CH=CH-CN + CH₂=CH₂

Par hydrogénation on obtient le nitrile en C11.
L'α-oléfine insaturée en C10 peut aussi être soumise à une coupure oxydante telle qu'une ozonolyse selon la réaction (simplifiée) suivante

CH₃-(CH₂)₇-CH=CH₂ + O₃ (O₂)→ CH₃-(CH₂)₇-COOH + HCOOH.

L'acide en C9 est estérifié en ester C9 qui peut, le cas échéant être transformé en nitrile C9. Comme indiqué précédemment, l'oléfine en C10 pourra être incorporée dans le mélange carburant. Elle pourra auparavant être hydrogénée.

L'acide érucique peut être soumis à une coupure oxydante telle que l'ozonolyse oxydante selon la réaction (simplifiée) suivante

CH₃-(CH₂)₇-CH=CH-(CH₂)₁₁-COOH + O₃ (O₂)→ CH₃-(CH₂)₉-COOH + COOH-(CH₂)₁₁-COOH.

L'acide C11 est transformé en ester C11.

En ce qui concerne les gras hydroxylés, ricinoléique (C18 :1 OH) et lesquérolique (C20 :1 OH) les processus appliqués sont légèrement différents.
Dans une variante préférée de traitement l'ester méthylique de l'acide ricinoléique est soumis à un craquage selon la réaction :

CH₃-(CH₂)₅-CHOH-CH=CH-(CH₂)₇-COOCH₃ (Δ)→ CH₃-(CH₂)₅-CHO + CH₂=CH-(CH₂)₈-COOCH₃

L'aldéhyde en C7 est aisément transformé en acide C7 par oxydation, puis en nitrile C7, la formation de la fonction aldéhyde étant une phase intermédiaire de la transformation de la fonction carboxyle en fonction nitrile.
L'aldéhyde C7 peut aussi être converti par action du méthanol en diméthylacétal qui peut être incorporé au mélange de carburant de la même manière que pour les oléfines ou alcanes évoqués précédemment.
L'ester ω-insaturé en C11, ester méthylique ω-undécylènique, issu du craquage peut être hydrolyse en acide ω-undécylènique. Une hydrogénation de l'ester ainsi obtenu permet d'obtenir l'ester en C11. Cet acide ω-undécylènique peut être transformé en nitrile w-undécylènique. En outre, en appliquant à cet ester ω-insaturé une réaction de métathèse avec l'acrylonitrile on obtient un nitrile-ester insaturé en C12.

Le même processus appliqué à l'acide lesquérolique conduit à la réaction :

CH₃-(CH₂)₅-CHOH-CH=CH-(CH₂)₉-COOCH₃ (Δ) → CH₃-(CH₂)₅-CHO + CH₂=CH-(CH₂)₁₀-COOCH₃

L'aldéhyde en C7 est aisément transformé en nitrile C7 ou converti en diméthylacétal comme indiqué ci-dessus.

Les procédés utilisés pour réaliser les transformations décrites ci-dessus sont bien connues de l'homme de l'art.
Les voies de nitrilation d'hydrocarbures fonctionnels (acides, aldéhydes, alcools) pour les transformer en fonction nitrile sont bien connues.
Un exemple de procédé industriel utilisant un acide gras comme matière première est celui de la fabrication de nitriles et/ou d'amines gras à partir d'acides gras extraits d'huiles végétales ou animales. Ce procédé qui est décrit dans l'encyclopédie Kirk-Othmer Vol 2, 4° Edition, page 411, date des années 1940. L'amine grasse est obtenue en plusieurs étapes. La première étape consiste en une méthanolyse ou une hydrolyse d'une huile végétale ou d'une graisse animale produisant respectivement l'ester méthylique d'un acide gras ou un acide gras. L'ester méthylique de l'acide gras peut ensuite être hydrolyse pour former l'acide gras. Enfin, l'acide gras est transformé en nitrile par réaction avec l'ammoniac (qui sera transformé finalement en amine par hydrogénation du nitrile ainsi obtenu).
Le schéma réactionnel de la synthèse des nitriles peut se résumer de la façon suivante. 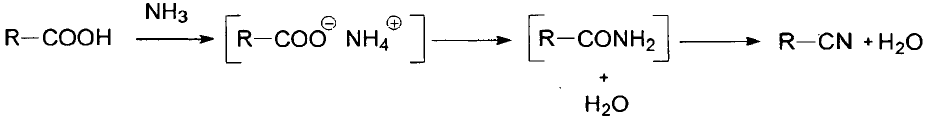 Il existe 2 types de procédés basés sur ce schéma réactionnel : un procédé batch en phase liquide et un procédé continu en phase vapeur.
Les procédés utilisent un agent de nitrilation choisi en général parmi l'ammoniac ou l'urée. et mettent en oeuvre un catalyseur oxyde métallique tel que ZnO ou autres.
On peut citer également les procédés décrits dans le brevet US 6,005,134 (Kao) visant un procédé catalytique à base d'oxyde de titane, les demandes japonaises N° 10-178414 et 11-117990 (Kao) décrivant l'utilisation d'oxyde de niobium et la demande japonaise N° 9-4965 visant un catalyseur à base de dioxyde de zirconium.
Enfin il faut relever le brevet US N° 4,801,730 qui décrit la synthèse de nitriles d'acides gras en présence d'ammoniac et d'un catalyseur à base de sulfonates organométalliques directement à partir des triglycérides constitutifs d'une graisse animale ou d'une huile végétale conduisant à un mélange de glycérol et de différents nitriles d'acides gras fonction de la nature de la charge. La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hérétogènes à base de métaux tels que Rhénium, Molybdène et Tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction vis-à-vis des réactions parasites telles que les « homo-métathèse » entre les réactifs mis en présence. Ils portent non seulement sur la structure des catalyseurs mais également sur l'incidence du milieu réactionnel et les additifs pouvant être introduits.
Dans les processus décrits ci-dessus, tout catalyseur de métathèse actif et sélectif pourra être utilisé. On utilisera cependant de préférence des catalyseurs à base de ruthénium.
La réaction de métathèse est généralement conduite à une basse température comprise entre 20 et 100°C.
Ces processus sont également décrits dans la demande WO08104722 au nom du déposant.

L'une des étapes possible des procédés de transformation des acides gras insaturés est la coupure par l'attaque au niveau de la double liaison de la molécule afin d'atteindre une division de celle-ci avec formation d'au moins une fonction aldéhyde ou acide terminale. Il s'agit là d'une coupure oxydante de la double liaison de la molécule. Cette coupure peut être réalisée au moyen d'oxydants forts tels que KMnO₄, le chlorochromate d'ammonium, l'eau oxygénée et plus particulièrement l'ozone d'où le terme d'ozonolyse largement employé. Elle peut être réalisée par craquage mais uniquement dans le cas où un radical hydroxyle est localisé en α e la double liaison.
On peut se référer au sujet de l'ozonolyse et plus largement de la coupure oxydante aux publications ci-après « Organic Chemistry » de L.G. Wade Jr. 5th Edition Chapter 8 Reactions of Alkenes, l'article de G.S. Zhang et al. Dans Chinese Chemical Letters, Vol 5, N°2, pp105-108 de 1994, l'article « Aldehydic Materials by the Ozonization of Vegetable Oils » Vol. 39 pages 496-500 , Journal of the American Oil Chemists Society. Il est fait référence au mécanisme réactionnel dit de « Criegee » marqué par la formation d'un ozonide.
Il apparaît que la mise en oeuvre de cette coupure oxydante est fonction des objectifs recherchés, soit l'ozonolyse oxydante conduisant à la formation de la fonction acide, soit l'ozonolyse réductrice si l'on cherche à s'arrêter au stade aldéhyde. Dans les processus décrits ci-dessus on choisira la coupure oxydante si le but est la formation de la fonction acide ou d'une fonction nitrile.
Le procédé de craquage des acides ricinoléique et lesquérolique est réalisé conformément au procédé industriel décrit dans l'ouvrage « Les Procédés de Pétrochimie » de A. Chauvel et al. paru aux Editions TECHNIP (1986) visant la synthèse de l'acide amino-11-undécanoique qui comporte plusieurs étapes.
La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part l'undécylénate de méthyle. Ce dernier est passé en forme acide par hydrolyse.
La transestérification au méthanol permet d'obtenir l'ester méthylique comme décrit dans la demande de brevet FR2918058. Le triglycéride de l'acide ricinoléique (huile de .ricin) est transestérifié par un excès de méthanol en présence de méthylate de sodium.
L'ester est alors vaporisé à 225 °C et ensuite mélangé avec de la vapeur d'eau surchauffée (620°C). La réaction est brève, une dizaine de secondes environ. On procède ensuite à la purification de l'undécénoate de méthyle, tout d'abord par refroidissement du milieu qui permet l'extraction de l'eau puis par une série de distillations permettant la séparation de l'ester et des sous-produits de la réaction.
Le mélange est craqué en présence de vapeur d'eau à une température de 620 °C. Le taux de conversion de l'ester méthylique est de 70 %. On sépare ensuite la fraction légère à pression atmosphérique, et celle principalement constituée d'heptanaldéhyde par distillation à pression réduite. Ensuite on distille la fraction constituée majoritairement de l'ester méthylique de l'acide undécylénique, sous un vide de 0,01 atm. Finalement, on distille la fraction riche en ester méthylique des acides oléique et linoléique de la fraction contenant l'ester méthylique de l'acide ricinoléique non converti, qui est éventuellement recyclée à l'étape de craquage. La fraction légère est constituée majoritairement des esters méthyliques des acides oléique et linoléique.

On peut utiliser toutes les plantes dont les graines donnent les acides gras insaturés supérieurs à 14 atomes de carbone. Cependant, l'huile de ricin qui contient plus de 80% poids d'acide ricinoléique et l'huile de Jatropha curcas qui contient de 30 à 50% poids en acide oléique sont des huiles non alimentaires qui présentent un intérêt important dans le débat actuel carburant versus alimentaire. De plus ces plantes ont des rendements par hectare particulièrement élevés qui les rendent très attractives. Elles peuvent aussi pousser dans des terrains très difficiles et dans des conditions de faibles pluviométries, là où peu de plantes alimentaires peuvent être cultivées, ce qui limite aussi les compétitions avec les applications alimentaires.

Les carburants aviation de l'invention sont illustrés par les exemples suivants.

### Exemple 1 : Préparation du nitrile de l'acide oléique

On utilisera dans ce cas une coupe d'acide oléique commerciale, contenant 76,8 % d'acide oléique. La coupe acide sera distillée sous vide afin de concentrer la fraction oléique jusqu'à une teneur de 85 %.

La préparation de nitrile est une opération classique industrielle pour la préparation d'amines grasses.

Une charge classique de 25 kg de ZnO comme catalyseur pour 40 Tonnes d'acide gras (soit 0,0625 %) sera utilisée. On opèrera avec un débit d'ammoniac de 1000 Nm³/h (soit 0,417 l/mn.kg). La température du réacteur sera augmentée progressivement de environ 1°C/mn de 160 °C jusque 305 °C. On commencera l'injection d'ammoniac à partir de 160 °C. On maintiendra la réaction sous ces conditions pendant environ 13 heures, jusqu'à ce que l'indice d'acidité soit inférieur ou égal à 0,1 mg de KOH/g. Pendant la réaction la température du déflegmateur sera maintenue à 130 °C.

La transformation en nitrile sera suivie d'une distillation simple où sera réalisée la purification du produit brut sur colonne de distillation de type Vigreux, en procédant à un étêtage du distillat. L'indice d'acide du nitrile obtenu sera de 0,030 mg KOH/g, l'indice d'iode sera de 98 g l₂/100 g, le rendement de distillation de 90 %.

Le produit final contiendra environ 85 % d'oléonitrile, 10 % de nitrile de l'acide stéarique, et 4 % de nitrile de l'acide palmitique.

### Exemple 2 : Ethénolyse de l'oléonitrile

L'oléonitrile de l'exemple précédent sera converti en décène-1 et nitrile décénoique par éthénolyse en présence du catalyseur Ruthenium, [1,3-bis(2,4,6-trimethylphenyl)-2-imidazolidinylidene]dichloro[[2-(1-methylethoxy)phenyl]methylene] CAS [301224-40-8] dont la formule est donnée ci-dessous. On opèrera dans du toluène à une concentration de 0,05 M, avec 0,5 % de catalyseur, à 50 °C. Après 22 heures, la conversion sera de plus de 99 %, et la sélectivité calculée de 42 % en décène et de 46 % en décènenitrile.

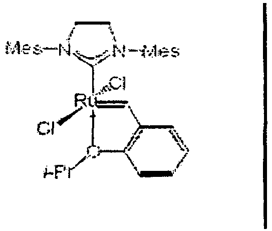

Le mélange ainsi obtenu aura une masse volumique de 0,78 g/ml, un point de trouble de - 50 °C. Il pourra être incorporé au kérosène selon les termes de l'invention.

### Exemple 3 : préparation du nitrile de l'acide undécylénique

On utilisera de l'acide undécylénique en provenance de l'usine Arkema de Marseille St Menet. Celui ci est préparé par hydrolyse de l'ester méthylique de l'acide undécylénique.
On procédera comme dans l'exemple 1 précédent. Cependant, le plus faible point d'ébullition de l'acide génèrera un reflux important qui ne permet pas d'atteindre 300 °C. Au bout de 12 heures la réaction ne sera pas encore complète car à poids égal il y a plus de moles à convertir. L'acidité résiduelle est de 0,2 % après 18 heures.
Après 21 heures de réaction à 264 °C, l'indice d'acidité final sera de 0,17 mg KOH/g. La purification du produit brut sera effectuée par distillation comme précédemment. La distillation sera effectuée sous un vide de 40 mBars, avec un rendement de distillation de 83 %.
L'analyse RMN du produit montrera que la double liaison du nitrile undécylénique s'est en partie déplacée (réaction d'isomérisation au cours de la réaction). Le produit final contiendra 96 % de 10-undécénenitrile, et 4 % d'isomères (où la double liaison C=C n'est plus terminale).

Le mélange ainsi obtenu aura une masse volumique de 0,83 g/ml, un point d'ébullition de 270 °C et pourra être incorporé en partie au kérosène selon les termes de l'invention.

### Exemple 4 : préparation de l'ester méthylique de l'acide undécylénique.

L'ester méthylique de l'acide 10-undécylénique sera synthétisé selon le procédé décrit précédemment (Les Procédés de Pétrochimie » de A. Chauvel et al).

La fraction légère issue de la dernière distillation sera constituée majoritairement des esters méthyliques des acides oléique et linoléique. Cette fraction qui représentera environ 15 % de l'huile de ricin, pourra être incorporée en tout ou partie aux côtés de l'ester méthylique de l'acide 10-undécylénique à la coupe kérosène.

Le mélange ainsi obtenu, contenant l'ester méthylique de l'acide undécylénique, et les esters méthyliques des acides oléiques et linoléiques aura une masse volumique de 0,88 g/ml, un point de trouble de - 20 °C. Il pourra être incorporé en partie au kérosène selon les termes de l'invention.
L'ester méthylique de l'acide 10-undécylénique utilisé seul, pourra être incorporé en plus grandes quantités dans le kérosène.

Exemple 5 : préparation de l'ester méthylique de l'acide 9-décénoique par éthénolyse Cet exemple illustre l'éthénolyse de l'oléate de méthyle. On utilisera pour cette réaction le catalyseur complexe [RuCl₂(=CHPh)(IMesH₂)(PCy₃)] dont la formule (A) est donnée ci-dessous. La réaction sera effectuée dans CH₂Cl₂, à une concentration de 0,05 M d'oléate de méthyle et 0,2 M d'éthylène à une température de 55°C, et pendant 6 heures, en présence du catalyseur à une concentration de 5 % mole par rapport à l'oléate de méthyle. Les rendements sont déterminés par analyse chromatographique. Le rendement en 9-décénoate de méthyle CH₂=CH-(CH₂)₇-COOCH₃ et en 1-décène sera de 55 % molaire.

### Catalyseur de formule (A)

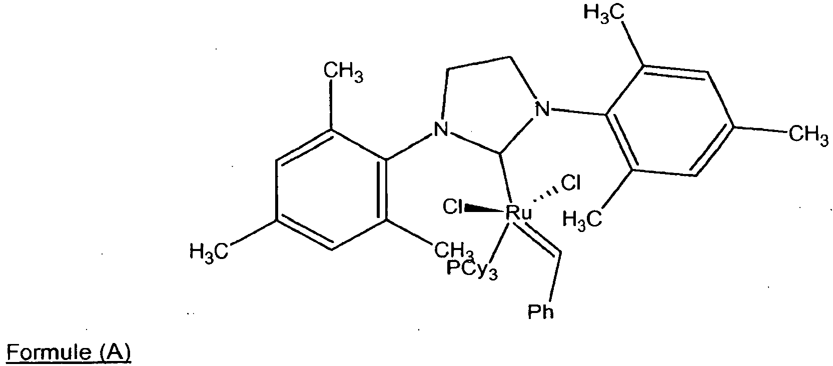

### Exemple 6 : préparation de l'ester méthylique de l'acide 9-décénoique par éthénolyse

Les expériences seront réalisées sous atmosphère d'Argon dans une boite à gants. L'ester méthylique de l'acide oléique (15 g) purifié par filtration sur alumine activée sera chargé dans un réacteur en verre agité. Une solution de 1000 ppm (teneur relative à l'ester méthylique) du catalyseur de métathèse (référence 57972-6, CAS [172222-30-9] acheté chez Sigma-Aldrich) sera préparée dans le dichlorométhane et sera ajoutée à l'oléate de méthyle. Le réacteur sera équipé d'une sonde de pression et d'un port d'introduction. Le système fermé sera retiré de la boite à gants et connecté à la ligne d'éthylène. Le réacteur sera alors purgé 3 fois avec de l'éthylène, puis pressurisé à 10 atm et placé dans un bain d'huile à 40°C, pendant 2 heures. Avant analyse chromatographique, la réaction sera stoppée en ajoutant une solution 1 M de tris-hydroxymethylphosphine dans l'isopropanol. Les échantillons seront alors chauffés pendant 1 h à 60 °C, dilués dans de l'eau distillée et extraits avec de l'hexane avant analyse par chromatographie en phase gazeuse. L'analyse révèlera un rendement en décène-1 de 30 %, et en 9-décénoate de méthyle de 30 %.

Le mélange d'ester méthylique d'acide décénoique et de 1-décène pourra être utilisé directement comme coupe incorporable au kérosène. Il sera aussi possible de laisser une partie de l'ester méthylique de l'acide oléique dans le mélange, ce qui signifie qu'il n'est pas nécessaire de rechercher une pureté élevée pour la coupe 1-décène plus ester méthylique de l'acide 10-undécylénique.

Un mélange équimolaire de méthyldécénoate et de 1-décène ainsi obtenu aura une masse volumique de 0,81 g/ml, un point de trouble de - 52 °C. Il pourra être incorporé au kérosène selon les termes de l'invention.

### Exemple 7 : préparation de l'ester méthylique de l'acide nonanoïque par coupure oxydante

1 kg d'huile oléique de tournesol (82 % d'acide oléique, 10 % d'acide linoléique) sera placé dans un réacteur avec 5 g d'acide tungstique et 50 g d'huile hydroxylée provenant d'une précédente synthèse. La température sera augmentée jusque 60-65 °C, puis 280 ml d'une solution d'eau oxygénée à 50 % seront ajoutés en 3 heures, tout en balayant le réacteur avec de l'azote pour limiter la présence d'eau dans le réacteur, et donc la dilution de l'eau oxygénée.

Une fois l'addition de l'eau oxygénée terminée, la réaction sera poursuivie pendant 3 heures. Le mélange formé à l'issue de l'étape précédente sera placé dans un autoclave agité. 300 g d'une solution aqueuse d'acétate de cobalt à 1 % (soit 0,4 % mol de Co par rapport au diol produit à l'étape précédente) seront ajoutés. La température sera ajustée à 70 °C, et le réacteur sera mis sous pression d'air à 12 bars. Le début de la réaction sera constaté par l'augmentation de température du mélange à cause de l'exothermie de la réaction. La réaction durera alors 8 heures.

Ensuite, la séparation de la phase aqueuse sera faite à chaud. La phase aqueuse sera séparée de la phase organique. La phase aqueuse, récupérable, contiendra les catalyseurs des étapes précédentes. La phase organique de l'huile oxydée, contiendra les triglycérides de l'acide azélaique produit par la réaction, en mélange avec l'acide pélargonique. La phase organique distillée permettra la récupération d'une fraction de 360 g d'acide pélargonique et autres acides gras courts.

L'acide sera ensuite estérifié en présence de méthanol

### Exemple 7bis : préparation de l'acide nonanoïque par ozonolyse

Cet exemple illustre la coupure oxydante de l'acide oléique pour donner l'acide nonanoïque par ozonolyse oxydante.

De l'ozone obtenue par un générateur d'ozone Welsbach T-408, sera bullée dans 25 ml de pentane jusqu'à ce qu'une couleur bleue soit observée. La solution de pentane sera maintenue à -70°C. avec un bain d'acétone-carboglace. 20 mg d'acide oléique, dissous dans 5 ml de pentane refroidis à 0 °C seront ajoutés à la solution d'ozone. L'excès d'ozone sera ensuite éliminé et la couleur bleue disparaîtra. Après 5 minutes, le pentane sera évaporé par un flux d'azote sec. Pendant cette étape la température de la solution sera maintenue inférieure à 0°C. Après évaporation du pentane, 3 ml de méthanol refroidis à -70 °C seront ajoutés au réacteur, tout en le réchauffant pour permettre la dissolution de l'ozonide. Pour effectuer la conversion de l'ozonide en acide, on commencera par élever la température à environ 60 °C. Quand la réaction de décomposition de l'ozonide démarre, elle s'accompagnera d'une élévation de la température. On ajoutera en continu un flux d'oxygène, pour maintenir la température et pour oxyder directement les produits issus de la décomposition de l'ozonide. On procédera en 4 heures pour limiter la formation de produits de dégradation. Il est important de maintenir la température de réaction légèrement au-dessus de la température de décomposition de l'ozonide au cours de cette étape. Une température de 95 °C sera utilisée dans cet exemple.

On obtiendra 6 mg d'acide de formule CH₃-(CH₂)₇-COOH.

### Exemple 8 : estérification de l'acide nonanoïque

L'acide nonanoïque préparé selon l'exemple 6 sera estérifié par le méthanol avec un excès stoechiométrique de 100, en présence d'acide sulfurique comme catalyseur (2 %). La réaction sera effectuée à reflux, pendant 2 heures. A la fin de la réaction l'ester méthylique de l'acide nonanoïque sera isolé par extraction au solvant, neutralisation des acides résiduels, suivies d'un lavage.

L'ester méthylique ainsi obtenu aura une masse volumique de 0,87 g/ml, un point de trouble de - 35 °C. Il pourra être incorporé en partie au kérosène selon les termes de l'invention.

Ainsi, dans des modes de réalisation préférés de l'invention,
- l'ester choisi est l'ester méthylique de l'acide 10-undécylénique ou undécanoïque.
- le nitrile choisi est le nitrile de l'acide décanoïque ou de l'acide 9-décénoique.
- l'ester choisi est le nonanoate de méthyle.
- le nitrile choisi est l'undécanitrile ou le 10-undécénitrile.

## Revendications

1. Carburant aviation **caractérisé en ce qu'**il comprend de 20 à 99 % poids d'une fraction d'un composé, obtenu par transformation chimique à partir d'acides gras mono insaturés éventuellement hydroxylés, naturels et renouvelables de longueur de chaîne au moins égale à 14 atomes de carbone, choisi parmi les nitriles et les esters d'acides gras moyens comportant de 7 à 12 atomes de carbone par molécule et de 1 à 80 % poids d'un kérosène issu du pétrole répondant aux spécifications mondiales des carburants aviation, et **en ce que** les esters d'acides gras moyens comportent un nombre impair d'atomes de carbone ou qu'ils comportent 10 et 12 atomes de carbone et sont ω-insaturés.

2. Carburant selon la revendication 1, **caractérisé en ce que** la fraction du composé sera constituée d'un mélange d'au moins un des composés nitrile et/ou ester et d'au moins une oléfine et/ou alcane comportant de 6 à 13 atomes de carbone par molécule, d'origine naturelle, issus de transformations chimiques analogues à celle appliquée auxdits acides gras mono insaturés éventuellement hydroxylés, naturels et renouvelables.

3. Carburant aviation selon la revendication 2 **caractérisé en ce que** la part de l'oléfine et/ou alcane dans le mélange constituant la fraction du composé représentera de 10 à 50 % poids dudit mélange.

4. Carburant selon l'une des revendications 1 à 3 **caractérisé en ce que** le nitrile gras comportant 12 atomes de carbone est ω-insaturé.

5. Carburant selon la revendication 1 **caractérisé en ce que** l'ester choisi est l'ester méthylique de l'acide 10-undécylénique ou undécanoïque.

6. Carburant selon l'une des revendications 1 à 3 **caractérisé en ce que** le nitrile choisi est le nitrile de l'acide décanoïque ou de l'acide 9-décénoique.

7. Carburant selon la revendication 1 **caractérisé en ce que** l'ester choisi est le nonanoate de méthyle.

8. Carburant selon l'une des revendications 1 à 3 **caractérisé en ce que** le nitrile choisi est l'undécanitrile ou le 10-undécénitrile.

## Patentansprüche

1. Flugzeugtreibstoff, **dadurch gekennzeichnet, dass** er 20 bis 99 Gew.-% einer Fraktion einer durch chemische Umwandlung aus natürlichen und erneuerbaren gegebenenfalls hydroxylierten einfach ungesättigten Fettsäuren mit einer Kettenlänge von mindestens 14 Kohlenstoffatomen erhaltenen Verbindung, die aus Nitrilen und Estern mittlerer Fettsäuren mit 7 bis 12 Kohlenstoffatomen pro Molekül ausgewählt ist, und 1 bis 80 Gew.-% eines Kerosins, das sich aus einem den globalen Spezifikationen für Flugzeugtreibstoffe entsprechenden Öl ergibt, umfasst und dass die Ester mittlerer Fettsäuren eine ungerade Zahl von Kohlenstoffatomen enthalten oder 10 und 12 Kohlenstoffatome enthalten und ω-ungesättigt sind.

2. Treibstoff nach Anspruch 1, **dadurch gekennzeichnet, dass** die Fraktion der Verbindung aus einem Gemisch von mindestens einer der Nitril- und/oder Esterverbindungen und mindestens einem Olefin und/oder Alkan mit 6 bis 13 Kohlenstoffatomen pro Molekül natürlichen Ursprungs, das sich aus analogen chemischen Umwandlungen wie den auf die natürlichen und erneuerbaren gegebenenfalls hydroxylierten einfach ungesättigten Fettsäuren angewandten Umwandlungen ergibt, besteht.

3. Flugzeugtreibstoff nach Anspruch 2, **dadurch gekennzeichnet, dass** der Anteil des Olefins und/oder Alkans in dem Gemisch, das die Fraktion der Verbindung ausmacht, 10 bis 50 Gew.-% des Gemischs beträgt.

4. Treibstoff nach einem der Ansprüche 1 bis 3, **dadurch gekennzeichnet, dass** das 12 Kohlenstoffatome enthaltende Fettnitril ω-ungesättigt ist.

5. Treibstoff nach Anspruch 1, **dadurch gekennzeichnet, dass** es sich bei dem gewählten Ester um den Methylester von 10-Undecylen- oder Undecansäure handelt.

6. Treibstoff nach einem der Ansprüche 1 bis 3, **dadurch gekennzeichnet, dass** es sich bei dem gewählten Nitril um das Nitril von Decansäure oder 9-Decensäure handelt.

7. Treibstoff nach Anspruch 1, **dadurch gekennzeichnet, dass** es sich bei dem gewählten Ester um Nonansäuremethylester handelt.

8. Treibstoff nach einem der Ansprüche 1 bis 3, **dadurch gekennzeichnet, dass** es sich bei dem gewählten Nitril um Undecannitril oder 10-Undecennitril handelt.

## Claims

1. Aviation fuel **characterised in that** it comprises from 20 to 99% by weight of a fraction of a compound, obtained by chemical conversion starting from natural and renewable, optionally hydroxylated, monounsaturated fatty acids with a chain length at least equal to 14 carbon atoms, chosen from nitriles and esters of medium fatty acids comprising from 7 to 12 carbon atoms per molecule, and from 1 to 80% by weight of a kerosene resulting from oil meeting the world specifications for aviation fuels, and **in that** the esters of medium fatty acids comprise an odd number of carbon atoms or **in that** they comprise 10 and 12 carbon atoms and are ω-unsaturated.

2. Fuel according to Claim 1, **characterized in that** the fraction of the compound will be composed of a mixture of at least one of the nitrile and/or ester compounds and of at least one olefin and/or alkane comprising from 6 to 13 carbon atoms per molecule, of natural origin, resulting from chemical conversions analogous to that applied to said natural and renewable, optionally hydroxylated, monounsaturated fatty acids.

3. Aviation fuel according to Claim 2, **characterized in that** the portion of the olefin and/or alkane in the mixture constituting the fraction of the compound will represent from 10 to 50% by weight of said mixture.

4. Fuel according to one of Claims 1 to 3, **characterized in that** the fatty nitrile comprising 12 carbon atoms is ω-unsaturated.

5. Fuel according to Claim 1, **characterized in that** the chosen ester is the methyl ester of 10-undecylenic or undecanoic acid.

6. Fuel according to one of Claims 1 to 3, **characterized in that** the chosen nitrile is the nitrile of decanoic acid or 9-decenoic acid.

7. Fuel according to Claim 1, **characterized in that** the chosen ester is methyl nonanoate.

8. Fuel according to one of Claims 1 to 3, **characterized in that** the chosen nitrile is undecanenitrile or 10-undecenenitrile.
